# FASCICULE DE BREVET EUROPEEN

(11) **EP 1 136 074 B1**
(45) Date de publication et mention de la délivrance du brevet: **23.07.2003**
(21) Numéro de dépôt: 01490011.2
(22) Date de dépôt: 20.03.2001
(51) Int. Cl.: A61K 35/78, A61K 7/26, A61P 1/00

(54) **Procédé d'extraction par un solvant de composés actifs de la chicorée**
Verfahren zur Lösungsmittelextraktion von aktiven Verbindungen aus Zichorie
Method of solvent extraction of active compounds from chicory

(30) Priorité: 22.03.2000 FR 0003669
(43) Date de publication de la demande: 26.09.2001
(73) Titulaire: Finaler, 59310 Orchies (FR)
(72) Inventeur: Hermand, Olivier, 59370 Haubourdin (FR)
(74) Mandataire: Hennion, Jean-Claude

(56) Documents cités:
- EP-A- 0 126 513
- DATABASE FSTA [en ligne] INTERNATIONAL FOOD INFORMATION SERVICE (IFIS), FRANFURT/MAIN, DE; MI-JUNG HONG ET AL: "Changes in browning characteristics of chicory roots by roasting processes." Database accession no. 1999-00-h0565 XP002156081 & JOURNAL OF THE KOREAN SOCIETY OF FOOD SCIENCE AND NUTRITION 27 (4) 591-595 1998 CORRESPONDENCE (REPRINT) ADDRESS, JOONG-HO KWON, DEP. OF FOOD SCI. & TECH., KYUNGPOOK NAT. UNIV., TAEGU 702-701, KOREA,

## Description

La présente invention concerne un procédé d'extraction par un solvant de composés actifs de la racine de chicorée et notamment de la chicorée torréfiée. La présente invention concerne également l'extrait obtenu par ce procédé et qui présente des propriétés particulièrement intéressantes notamment dans le domaine de l'hygiène buccale. La présente invention concerne encore un dentifrice et un bain de bouche contenant l'extrait de l'invention.

La racine de la chicorée sauvage ou à café encore appelée Cichorium Intybus Linn (abrégé dans la suite du présent texte en C.I.L.) est utilisée depuis l'Antiquité dans le domaine alimentaire et pour ses propriétés médicinales.

Dans le domaine alimentaire, la racine de la chicorée est souvent utilisée après torréfaction. En revanche, dans la domaine pharmaceutique, ce sont des racines séchées à l'air chaud qui sont presque exclusivement utilisées.

En 1983, Patel et Venkatakrishna ont montré les propriétés de l'extrait de racine de chicorée torréfiée pour la prévention de la plaque dentaire et le traitement des gingivites. L'extrait utilisé par ces auteurs est obtenu par extraction à chaud à partir de poudre brute de racine de chicorée séchée et torréfiée en présence d'eau ou d'éthanol dans un extracteur de Soxhlet.

L'extrait ainsi obtenu se présente sous la forme d'un liquide visqueux de couleur noire. Cet extrait de chicorée utilisé en massage permet de diminuer dans une large mesure l'inflammation gingivale, présente des propriétés antimicrobiennes et s'avère être un inhibiteur efficace de la plaque dentaire.

La présente invention propose un procédé d'extraction par un solvant de composés actifs de la chicorée différent de celui précédemment décrit.

Selon le procédé d'extraction par un solvant de l'invention, de manière caractéristique, on fait macérer de la chicorée dans un solvant, à une température comprise entre 0°C et 6°C, pendant une durée supérieure ou égale à 12 heures et l'on filtre le jus de macération avec un seuil de coupure d'une valeur au moins égale à 0,22 µm.

La filtration à un tel seuil de coupure permet d'obtenir à partir d'un jus de macération de chicorée quelconque, un extrait limpide et actif ce qui n'est pas évident du fait que les composés actifs de la chicorée ne sont pas identifiés à ce jour. Cependant, il n'est pas exclu, notamment si la limpidité n'est pas rédhibitoire de réaliser la filtration avec un seuil de coupure plus élevé ou de réaliser la séparation de l'extrait et des résidus solides par d'autres techniques de séparation.

Il est également possible selon l'invention, en fonction de la quantité de matières solides extraites, de procéder à une ou plusieurs étapes de séparation solide-liquide, éventuellement par filtration, avant de mettre en oeuvre l'étape de filtration du jus de macération avec un seuil de coupure d'une valeur au moins égale à 0,22µm.

Sachant que la solubilité des composés contenus dans la chicorée, par exemple l'inuline, (et leurs coefficients de diffusion) augmente avec la température, il est donc tout à fait surprenant qu'il soit possible d'extraire des composés actifs de la racine de chicorée aux températures prévues par le procédé de l'invention. Les températures basses du procédé d'extraction de l'invention permettent de plus une bonne conservation de l'extrait obtenu, sans ajout de conservateur puisqu'aux températures d'extraction du procédé de l'invention, les bactéries se développent peu. De plus, le procédé de l'invention évite toute dégradation thermique de la chicorée qui pourrait éventuellement dénaturer les composés actifs.

De même, la Demanderesse a également mis en évidence qu'une filtration jusqu'à un seuil de coupure égal à 0,22 µm permet d'obtenir un extrait, comprenant tous les composés actifs, d'une couleur et d'une limpidité optimale pour son utilisation ultérieure.

Selon l'invention, la quantité de chicorée torréfiée utilisée est telle que le mélange solvant plus chicorée avant macération contient au moins 5% et de préférence 7,4% de matière sèche. De telles quantités permettent en effet d'obtenir des extraits présentant ou susceptibles de présenter les propriétés pharmacologiques (efficacité antimicrobienne et anti-inflammatoire) et physiques (trouble, pourcentage de matière sèche, couleur) optimales pour leur utilisation ultérieure.

La chicorée utilisée dans le cadre de la présente invention n'est pas limitée à une catégorie spécifique de chicorée. Il peut s'agir, par exemple, de chicorée séchée, torréfiée ou même de la chicorée soluble disponible dans le commerce. De préférence, dans le cas de la chicorée torréfiée, celle-ci est obtenue à l'issue d'une torréfaction dont la température finale n'excède pas 170°C. De plus hautes températures de torréfaction causent en effet des changements indésirables tels qu'une baisse de la fraction extractible et du pH ainsi qu'un goût faible et piquant.

Selon l'invention, la chicorée torréfiée peut se. présenter sous la forme de grains, de grains moulus, de poudre, de liquide ou de pâte. Néanmoins, pour optimiser la quantité de composés actifs qu'il est possible d'extraire selon le procédé de l'invention, il est préférable d'utiliser de la chicorée en poudre en sorte de maximiser la surface de contact entre la chicorée et le solvant.

On peut obtenir une limpidité de l'extrait suffisante si l'on effectue une filtration du jus de macération avec un seuil de coupure inférieur ou égal à 10µm.

Le solvant utilisé selon le procédé de l'invention n'est pas limité. Il peut s'agir de solvants protiques comme, par exemple, l'eau ou les alcools. De préférence, on utilise l'eau à la fois pour des questions de coût et d'innocuité ce qui s'avère intéressant pour les utilisations ultérieures de l'extrait obtenu selon le procédé de l'invention.

L'extrait obtenu selon la procédé de la présente invention peut notamment être utilisé pour la préparation d'une composition destinée à l'hygiène buccale. Il peut également être utilisé pour la préparation d'une composition cosmétique. En effet, l'extrait obtenu par le procédé de l'invention présente toutes les propriétés connues de l'extrait de chicorée obtenu par extraction à chaud, à savoir des propriétés antimicrobiennes et anti-inflammatoires sans en présenter la couleur noire qui en limite l'utilisation.

De préférence, le bain de bouche de l'invention contient une quantité comprise entre 8% et 12% et de préférence égale ou sensiblement égale à 10% en masse de l'extrait de chicorée obtenu selon le procédé de l'invention.

De même, le dentifrice de l'invention contient une quantité comprise entre 8% et 12% et de préférence égale ou sensiblement égale à 10% en masse de l'extrait de chicorée obtenu selon le procédé de l'invention.

La présente invention sera mieux comprise et ses caractéristiques et avantages apparaîtront mieux à la lecture de la description et des exemples de mise en oeuvre qui suivent.

### Exemple de préparation d'un premier extrait de chicorée de l'invention

La chicorée torréfiée utilisée dans cet exemple présente les caractéristiques physico-chimiques suivantes :

| | |
|---|---|
| couleur | 50 à 70 (color test Neuhaus) |
| matière sèche mesurée à 70°C | > 95% |
| granulométrie moyenne | 90% < 0,710 mm |
| pH en solution aqueuse à 10% | compris entre 4,0 et 4,5 |

et les caractéristiques bactériologiques suivantes :

| | |
|---|---|
| germes aérobies mésophiles | < 10/g |
| levures : | 0/ g |
| moisissures : | 0 / g |

100g de la chicorée en poudre définie ci-dessus ont été mélangés dans un réacteur calorifugé à de l'eau osmosée refroidie à 4°C. Le mélange est mis sous faible agitation pendant environ une minute afin de l'homogénéiser. La quantité d'eau utilisée est telle que la matière sèche du mélange eau + chicorée représente 7,4% en masse du mélange. Le mélange est maintenu à 4°C pendant 24 heures sans agitation. On effectue ensuite une première filtration en sorte d'éliminer les matières en suspension. Cette filtration est par exemple mise en oeuvre au moyen d'une toile de filtre presse présentant un seuil de coupure de 10-20µm. Cette première filtration peut également être mise en oeuvre par centrifugation ou par toute autre technique connue de séparation solide-liquide. Après cette première filtration on obtient un marc solide qui représente environ 18% en volume du mélange initial et dont le pourcentage de matière sèche est de 16,5%. Le jus de macération également obtenu représente 82% en volume du mélange initial et présente un taux de matière sèche de l'ordre de 6%. Ce jus de macération est dilué avec de l'eau osmosée afin de le ramener au volume initial d'eau mélangé à la chicorée. Après cette dilution, le taux de matière sèche du jus de macération se situe aux alentours de 4,5%.

On effectue alors une seconde filtration qui a principalement pour but d'obtenir un jus de macération limpide. Pour ce faire on utilise des plaques de cellulose du type K900, K300 ou S80 qui présentent un seuil de coupure moyen compris respectivement entre 8 et 10µm, 4µm et 6 µm. et 0,22µm. Toute autre technique analogue, comme la filtration membranaire peut également être utilisée. Le filtrat obtenu correspond à l'extrait de composés actifs de chicorée de la présente invention.

Cet extrait présente les caractéristiques suivantes :

| | |
|---|---|
| matière sèche totale | 4 à 5% |
| pH | 4 à 4,5 |
| cendres/matière sèche | 4,4 à 5,6% |
| protéines/matière sèche | 3,1 à 4,1% |
| coloration (mesurée à 420 nm) | 1,500 à 1,700 |
| trouble (mesuré à 600 nm) | 0,100 à 0,140 |
| glucose/matière sèche | 7,5 à 9,5% |
| fructose/matière sèche | 7,0 à 9,0 % |
| DP2/matière sèche | 14,0 à 16,0 % |
| inuline / matière sèche | 6 à 9% |

Dans le cas d'un extrait obtenu après centrifugation pour la séparation solide-liquide et filtration avec un seuil de coupure de 0,22µm, on obtient une valeur moyenne globale de la coloration mesurée à 420 nm comprise entre 0,800 et 1,700 et une valeur moyenne du trouble mesurée à 600 nm comprise entre 0,085 et 0,140.

Le fait de mettre en oeuvre l'étape de macération sans agitation permet d'influencer le bilan matière et la répartition liquide/solide. La Demanderesse a en effet noté que la teneur en inuline de l'extrait obtenu est plus faible sans agitation (inférieure à 37%) alors que la teneur en sucres libres de l'extrait est indépendante de l'agitation. Les résultats concernant l'influence de l'agitation sont regroupés dans les tableaux I à III annexés à la présente description.

L'activité antimicrobienne de l'extrait de l'invention a également été testée.

### Etude in vitro de l'activité antibactérienne d'un second exemple d'extrait de l'invention

200 g de C.I.L. sous forme séchée sont mécaniquement réduits en poudre et partagés en deux lots de 100 grammes chacun. Un premier lot dit CIC/SEC/ED est repris par 400 ml d'eau distillée soumis à agitation magnétique en chambre froide à 4°C pendant une nuit. Le mélange ayant beaucoup gonflé, 400 ml d'eau distillée sont rajoutés et l'extraction aqueuse est reprise. Après 24 heures, le mélange est filtré et le matériel retenu est abondamment lavé.

Un deuxième lot dit CIC/SEC/ALC est repris par 400 ml d'éthanol absolu (99%) est soumis à agitation magnétique en chambre froide à 4°C pendant une nuit. Le mélange est alors filtré et le matériel retenu est abondamment lavé à l'éthanol pur. Le filtrat alcoolisé est évaporé au rotavapeur Buchi sous vide à 50°C. Le résidu pâteux est repris par 30 ml d'eau distillée et mis en dialyse contre de l'eau distillée : la dialyse est poursuivie pendant quatre jours en renouvelant l'eau distillée de dialyse chaque jour. L'extrait est alors conservé en congélation à -34°C. Deux autres lots appelés respectivement CIC/GRI/ED et CIC/GRI/ALC ont été préparés conformément à ce qui précède à partir de chicorée grillée, c'est-à-dire torréfiée.

Chacun des extraits secs congelés en coquille est soumis à lyophilisation. La lyophilisation s'est avérée longue mais aucune décongélation précoce ou intempestive des échantillons n'a été observée.

A l'issue de la lyophilisation, les quantités de matière sèche ont été mesurées.

| | CIC/SEC/ED | CIC/GRI/ED | CIC/SEC/ALC | CIC/GRI/ALC |
|---|---|---|---|---|
| POIDS (g) | 7,96 | 14,36 | 0,4462 | 0,6742 |
| POIDS SEC DE L'EXTRAIT/200 G DE MATIERE PREMIERE(%) | 3,98 % | 7,18 % | 0,22 % | 0,33% |

Les extraits aqueux (CIC/SEC/ED et CIC/GRI/ED) sont repris par 100 ml d'eau distillée tandis que les extraits alcoolisés sont repris par 10 ml d'eau distillée. Une solution de chicorée soluble du commerce a également été préparée à raison de 10 g pour 100 ml de milieu de culture TGY. Le milieu TGY est ainsi défini :
- trypticase : 30 g
- extrait de levure : 10 g
- glucose : 27 mM
- tampon Tris : 100 mM
- acide chlorhydrique qsp : pH 7,5
- eau bi-distillée qsp : 1000 ml

Le milieu ainsi formé est réparti dans des tubes de 25 ml et stérilisé en autoclave.

Ce milieu TGY sert à la culture de Streptococcus mutans IM 2201 et Bacterionoma matruchotii IM 2219 issus tous deux de la collection de l'Institut Mérieux. Les cultures introduites dans le milieu TGY sont incubées à 37° C pendant 24 heures.

Par ailleurs, de la plaque dentaire humaine fraîchement récupérée sur patient fut également mise en culture dans le milieu liquide « cerveau-coeur ». Après 24 h de développement, cet isolat de plaque est repiqué sur le milieu décrit plus haut.

De même, Prevotella intermedia AIP N 161/79 (ex Bacteroides intermedius) et Porphyromonas gingivalis ATCC 33277 issus de la collection de l'institut Pasteur de Paris et de la collection américaine sont cultivés dans des conditions anaérobies pendant 24 heures et 48 heures à 37°C dans un milieu liquide ayant la composition suivante :
- trypticase : 30g
- extrait de levure : 20g
- cysteine : 0,5g
- hémine : 5 mg
- glucose : 180mM
- eau distillée qsp : 1000ml

Des milieux de culture test ont été préparés. Des tubes TGY de 25 ml reçoivent respectivement 0, 5 ml d'extrait chicorée séchée aqueux (CIC/SEC/D = 0,15 % p/v), 0,5 ml d'extrait chicorée torréfié aqueux (CIC/GRI/ED = 0,28 % p/v), 1ml de solution de chicorée du commerce (= 0,4 % p/v), 0,5 ml d'extrait chicorée torréfié alcoolique (CIC/GRI/ALC = 0,09 % p/v) et 0,5 ml d'extrait chicorée séchée alcoolisée (CIC/SEC/ALC =0,13 % p/v). Chaque tube de culture supplémenté en extrait aqueux contient un équivalent de 1g C.I.L. et de 10 g pour l'extrait alcoolique. Tous ces tubes sont introduits dans un autoclave pendant 30 minutes et conservés à 4°C. Il est à noter que ces concentrations sont compatibles avec les conditions d'utilisation de la chicorée en alimentation humaine.

A chacun des milieux tests précédemment décrits auxquels sont ajoutés des tubes témoins dépourvus de toute trace de chicorée sous quelque forme que ce soit, est incorporé dans des conditions stériles, 1 ml de milieu de culture âgé de 24 h, soit de Streptococcus mutans soit d'isolat de plaque bactérienne dentaire humaine.

Les études statistiques montrent l'activité de l'extrait de chicorée. Pour des temps s'échelonnant de 0 à 21 h, des prises de 3 ml sont effectuées dans chaque tube après agitation vortex. Chaque prise est soumise à centrifugation 500 x g pendant 5 mn à 4°C (ces conditions respectent l'intégrité des corps bactériens). Les surnageants sont soigneusement éliminés et à chaque culot sont ajoutés 2 ml d'eau distillée. Le tout est alors soumis à agitation vortex afin de redisperser les cellules bactériennes dans la phase aqueuse. La turbidité qui reflète la croissance bactérienne pour une culture donnée est mesurée pour chaque échantillon dans un spectrophotomètre Beckman DU5 (dispositif d'aspiration à la température régulée à 610 nm de longueur d'ondes).

Il ressort de ces expérimentations que les extraits de C.I.L de l'invention perturbent la croissance bactérienne pendant la phase logarithmique. Pour Streptococcus mutans IM 2209, l'effet le plus sensible se manifeste dans les premières heures de la croissance.

C'est la culture de Bacterionema matruchotii IM 2219 qui semble être la plus sensible aux extraits de C.I.L. de l'invention. L'effet inhibiteur est surtout manifeste dans la deuxième phase de croissance entre 28 et 48 heures avec toutes les formes de C.I.L. (séché aqueux, torréfié aqueux, séché alcoolique, torréfié alcoolique et chicorée du commerce).

La Demanderesse a également mis en évidence ce même effet inhibiteur sur la plaque bactérienne dentaire humaine mise en culture avec un ralentissement de la croissance globale manifeste dès la onzième heure, plus prononcé avec l'extrait aqueux de chicorée grillée. Les germes anaérobies testés sont eux aussi sensibles aux extraits de C.I.L. de l'invention.

Avec Prevotella Intermedia, cet effet est plus marqué avec les extraits aqueux mais reste significatif avec toutes les formes utilisées y compris avec la chicorée commerciale. En ce qui concerne Porphyromonas gingivalis, sa croissance est affectée à hauteur d'environ 10 à 20 %.

Il est à noter que les concentrations des extraits de C.I.L. de l'invention utilisés ici sont comparables aux taux habituellement consommés en alimentation humaine, soit pour une solution de milieu de culture de 25 ml, l'équivalent de 1 g de matière première (extrait aqueux) ou 10 g (extrait alcoolique).

En conclusion, l'extrait de C.I.L. de la présente invention réduit in vitro la croissance des espèces bactériennes les plus représentatives de la flore buccale. Leur croissance est ralentie en moyenne de 10 à 50 % selon les espèces, la nature des extraits et les temps de culture sur milieu liquide.

### Etude in vivo de l'activité antibactérienne du premier exemple d'extrait de l'invention

L'extrait de l'invention utilisé pour cette étude est obtenu après macération à 4°C pendant 24 heures sans agitation d'un mélange de chicorée torréfiée dans de l'eau osmosée (contenant 7,4 % en masse de matière sèche) et filtration du jus de macération ainsi obtenu avec un seuil de coupure compris entre 4µm et 10µm. La concentration en chicorée de cet extrait est telle qu'il contient environ 4,5 % en masse de matière sèche.

Cet extrait a été utilisé pour la préparation d'un bain de bouche et d'une pâte dentifrice qui contiennent chacun environ 10% en masse de cet extrait.

La pâte gingivale et le bain de bouche ainsi obtenus ont été comparés à une pâte gingivale à base de Sanguinarine, un bain de bouche à la chlorhexidine et deux placebos (bain de bouche et pâte gingivale).

La chlorhexidine est un composé communément employé actuellement en parodontologie ; c'est un antiseptique efficace utilisé dans de nombreux domaines de la médecine en raison de sa faible toxicité et de son large spectre antibactérien.

La sanguinarine est un antiseptique obtenu par extraction alcoolique à partir de la plante Sanguinaria canadensis originaire du nord Canada. On a noté in vivo une activité de la sanguinarine sur l'inflammation gingivale. Son utilisation est fréquemment suggérée en phase de maintenance du traitement parodontal en complément des mesures d'hygiène orale.

L'étude suivante a été menée sur 45 jours pour chaque patient sur une période s'étendant de septembre 1998 à octobre 1999 et a concerné 120 patients répondant aux critères d'inclusion. A chaque patient était attribué un dossier numéroté. Les patients étaient âgés de 35 à 65 ans et présentaient une parodontite d'adulte selon les critères de l'American Academy of Parodontology et au moins une poche parodontale supérieure à 3 mm confirmée par l'examen radiographique et clinique (critères d'inclusion)
Les patients ont été séparés arbitrairement en six groupes :
- chicorée bain de bouche,
- chicorée bain de bouche placebo,
- chicorée pâte gingivale,
- chicorée pâte gingivale placebo,
- pâte gingivale à la sanguinarine (PERIOGARD ®), bain de bouche à la chlorhexidine à 0,12 % (PAROEX ®).

Chaque groupe comprend 20 patients.
Au jour « J0 » de l'expérimentation, un détartrage supra gingival est effectué pour placer les patients dans des conditions d'hygiène initiale proche. Trois indices ont été relevés lors de l'étude sur six dents (16, 12, 24, 36, 32, 44) :
- indices de plaque (Silness et Loe 1964) -,
- indice gingival (Loe et Silness 1963 - GI -),
- indice de saignement (Mühlemann - HR - modifié en 1981).

Pour le groupe chicorée bain de bouche, la diminution des indices est observable entre J15 et J45 pour les indices de plaque, gingivale et les saignements. L'évolution des indices de J 15 à J 45 est de 0,93 à 0,53 (indice de plaque), 1,01 à 0,52 (indice gingival), 1,04 à 0,51 (indice de saignement). Ces diminutions semblent d'une amplitude comparable à celle obtenue avec le bain de bouche PAROEX®.

Pour le groupe utilisant un bain de bouche PAROEX ® ,les indices évoluent tous favorablement confirmant l'efficacité déjà prouvée de la chlorhexidine sur les paramètres cliniques de l'inflammation et la réduction de l'accumulation de la plaque bactérienne. L'évolution des indices de J 15 à J 45 est de 0,87 à 0,45 (indice de plaque), 0,96 à 0,58 (indice gingival), 0,97 à 0,62 (indice de saignement).

Concernant le groupe placebo bain de bouche, l'évolution des indices de J 15 à J 45 est de 0,69 à 0,60 (indice de plaque), 0,79 à 0,66 (indice gingival), 0,97 à 0,71 (indice de saignement). Le produit ne semble pas modifier de façon significative l'indice de plaque, a peu d'action sur l'indice gingival malgré l'effet du brossage et a réduit l'indice de saignement. L'étude statistique précisera si ces variations sont significatives.

Pour le groupe chicorée pâte gingivale, il a été observé une baisse globale sur les trois indices cliniques. L'évolution des indices de J 15 à J 45 est de 0,84 à 0,48 (indice de plaque), 0,90 à 0,46 (indice gingival), 0,97 à 0,51 (indice de saignement).

Pour le groupe PERIOGARD ®, l'évolution des indices J 15 à J 45 est de 0,60 à 0,49 (indice de plaque), 0,65 à 0,61 (indice gingival), 0,83 à 0,68 (indice de saignement).

Concernant le groupe placebo pâte gingivale, l'évolution des indices de J 15 à J 45 est de 0,80 à 0,81 (indice de plaque), 0,85 à 0,73 (indice gingival), 0,81 à 0,90 (indice de saignement). Les indices observés présentent des modifications faibles ou nulles entre J 15 et J 45 en fin d'étude. L'indice de saignement présente une augmentation sensible montrant qu'il s'agit bien d'un groupe placebo.

Les études statistiques effectuées (test de Student) montrent que les différences observées sont pour tous les indices significatives, très significatives, voire hautement significatives au jour J 45 dans le groupe « chicorée bain de bouche » et le groupe « chicorée pâte gingivale ». De même, les variations observées entre J 15 et J 45 sont comparables, voire supérieures (pâte gingivale) à celles observées avec les produits actifs utilisés en groupe contrôle.

Il est à noter que l'extrait de l'invention utilisé pour la préparation du bain de bouche et de la pâte dentifrice ci-dessus décrits présente une saveur et une couleur telles que notamment dans la proportion précédemment indiquée (10%), la pâte dentifrice et le bain de bouche obtenus ont tous une coloration beige pâle acceptable par le consommateur et un goût peu prononcé qualifié comme non gênant voire d'agréable par les patients utilisateurs. L'utilisation de l'extrait obtenu à chaud ne permet d'obtenir de tels résultats.

Par ailleurs, aucune coloration des dents ni des muqueuses jugales, labiales et gingivales des utilisateurs n'a été notée.

L'activité anti-inflammatoire de l'extrait de l'invention a également été testée in vitro.

### Etude in vitro de l'activité anti-inflammatoire d'un exemple d'extrait de l'invention

L'extrait utilisé pour cette étude correspond à l'extrait de l'invention obtenu dans l'exemple de préparation d'un premier extrait de chicorée décrit précédemment. Le but principal de l'essai est d'étudier les mécanismes d'action de la chicorée sur l'inflammation et l'effet sur la sécrétion d'interleukine 1 alpha (IL-1α) marqueur précoce de l'inflammation.

Le modèle in vitro utilisé sont des cultures de kératocytes gingivaux humains normaux. L' IL-1α a été mesurée dans les milieux d'incubation des kératocytes par dosage ELISA. Les effets de l'extrait de chicorée de l'invention ont été comparés à ceux observés en présence de dexaméthasone, utilisée comme produit de référence. L'extrait de chicorée testé dans des concentrations de 0,01 et 0,4% (P/V) montrait après 24 heures d'incubation, un effet qui peut être qualifié d'anti-inflammatoire dans des kératocytes gingivaux humains en culture. Les effets observés sont comparables voire supérieurs à ceux obtenus en présence de la dexaméthasone, produit anti-inflammatoire pharmacologique de référence.

Par ailleurs, La Demanderesse a confirmé que l' IL-1α augmentait la protéolyse de la matrice extra cellulaire des fibroblastes gingivaux humains en culture. Ainsi, l'extrait de chicorée de l'invention qui diminue la libération d'IL-1α à partir des cellules supérieures de la muqueuse buccale (les kératocytes), peut prétendre exercer une protection anti-protéolyse de la matrice extra cellulaire de la gencive en réponse à une réaction inflammatoire.

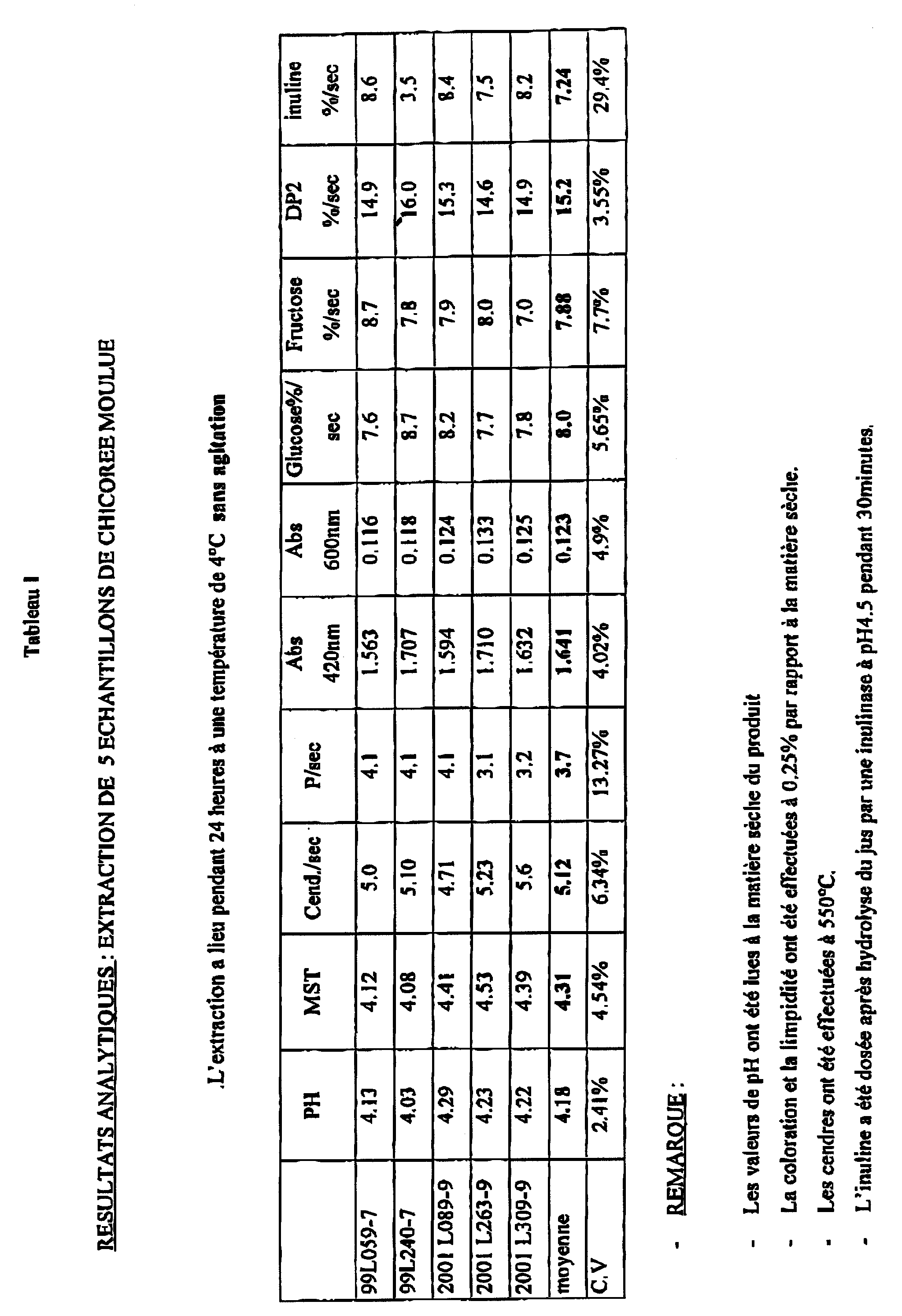

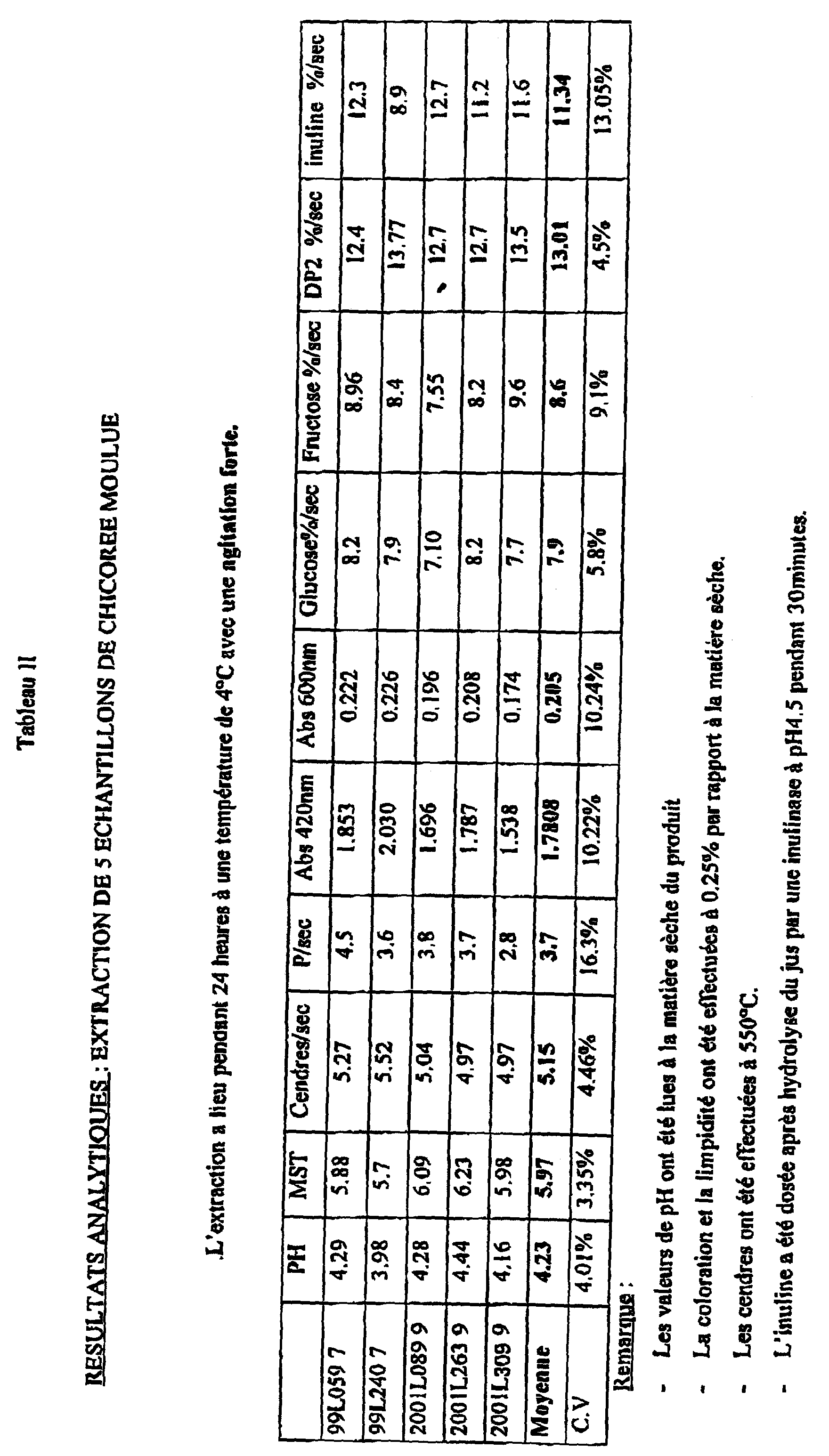

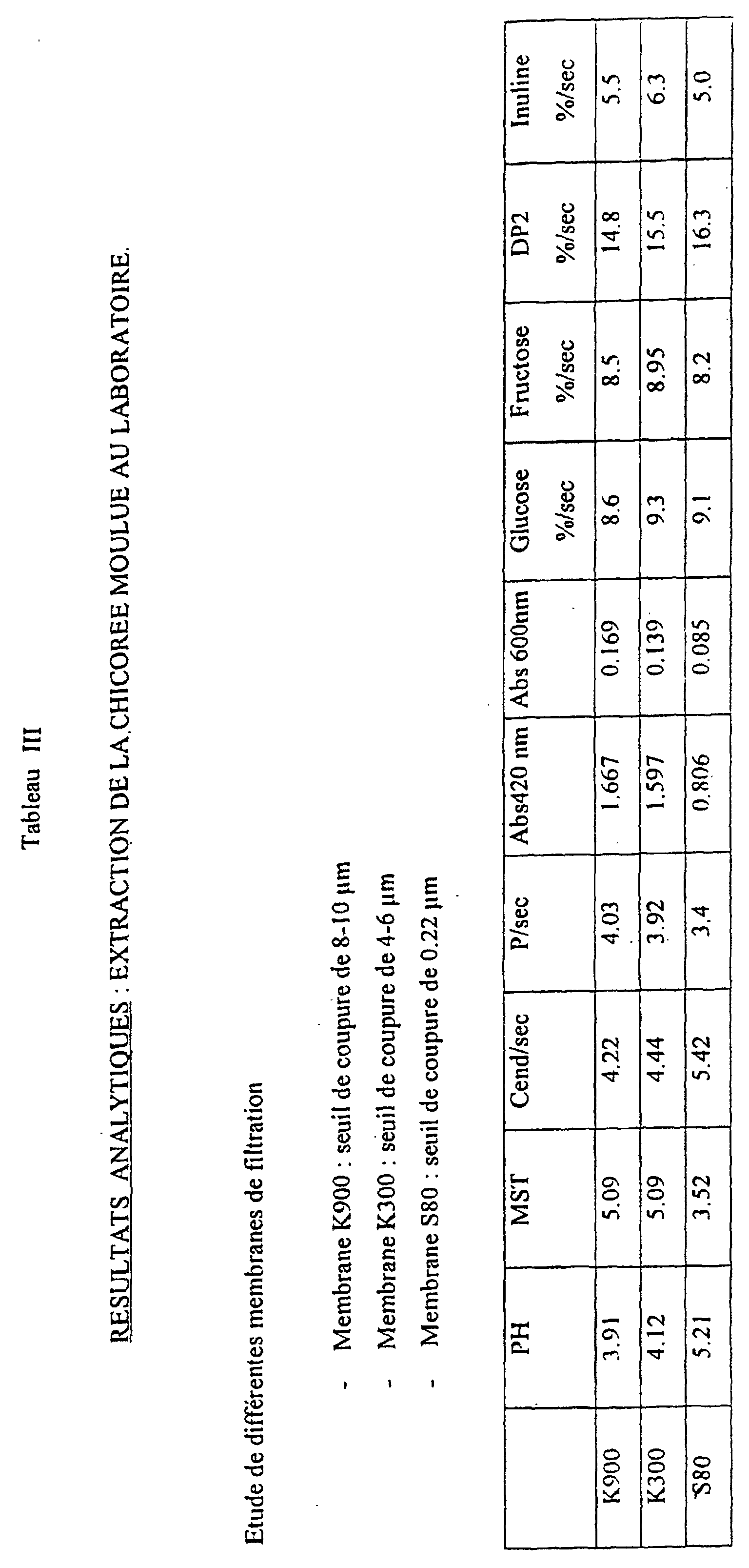

## Revendications

1. Procédé d'extraction par un solvant de composés actifs de la chicorée, **caractérisé en ce que** :
- on fait macérer de la chicorée dans ledit solvant, à une température comprise entre 0°C et 6°C, pendant une durée d'au moins douze heures ; et
- on filtre le jus de macération avec un seuil de coupure d'une valeur au moins égale à 0,22 µm.

2. Procédé selon la revendication 1, **caractérisé en ce que** la quantité de chicorée utilisée est telle que le mélange solvant plus chicorée contient au moins 5% et de préférence 7,4% de matière sèche.

3. Procédé selon l'une des revendications 1 ou 2, **caractérisé en ce que** l'on effectue préalablement à la filtration avec un seuil de coupure d'au moins 0,22µm une première filtration avec un seuil de coupure compris entre 10µm et 20µm.

4. Procédé selon la revendication 3, **caractérisé en ce qu'**après ladite première filtration on dilue le filtrat en sorte que la matière sèche dudit filtrat soit sensiblement égale ou égale à 4,5%.

5. Procédé selon l'une quelconque des revendications 1 à 4, **caractérisé en ce que** ladite chicorée est torréfiée avant macération.

6. Procédé selon l'une quelconque des revendications 1 à 5, **caractérisé en ce que** ladite chicorée est sous forme de poudre et **en ce que** l'on réalise un mélange homogène de la chicorée et dudit solvant, sous faible agitation.

7. Procédé selon l'une quelconque des revendications 1 à 6, **caractérisé en que** la phase de macération est mise en oeuvre sans agitation.

8. Procédé selon l'une des revendications 1 à 7, **caractérisé en ce que** l'on filtre le jus de macération avec un seuil de coupure inférieur ou égal à 10µm.

9. Procédé selon l'une quelconque des revendications 1 à 8, **caractérisé en ce que** ledit solvant utilisé est l'eau.

10. Procédé selon la revendication 9, **caractérisé en ce que** l'on fait macérer ladite chicorée à une température sensiblement égale ou égale à 4°C.

11. Procédé selon la revendication 10, **caractérisé en ce que** l'on fait macérer ladite chicorée pendant une durée sensiblement égale ou égale à 24 heures.

12. Utilisation de l'extrait de chicorée obtenu selon le procédé de l'une quelconque des revendications 1 à 11 pour la préparation d'une composition destinée à l'hygiène buccale.

13. Utilisation de l'extrait de chicorée obtenu selon le procédé de l'une quelconque des revendications 1 à 11 pour la préparation d'une composition cosmétique.

14. Bain de bouche contenant une quantité comprise entre 8% et 12% et de préférence égale ou sensiblement égale à 10% en masse de l'extrait de chicorée obtenu selon le procédé de l'une quelconque des revendications 2 à 11.

15. Dentifrice contenant une quantité comprise entre 8% et 12% et de préférence égale ou sensiblement égale à 10% en masse de l'extrait de chicorée obtenu selon le procédé de l'une quelconque des revendications 2 à 11.

## Patentansprüche

1. Verfahren zum Extrahieren von aktiven Verbindungen (Wirkstoffen) aus Zichorie mit einem Lösungsmittel, **dadurch gekennzeichnet, dass** man
- die Zichorie in dem Lösungsmittel bei einer Temperatur zwischen 0 und 6 °C für eine Zeitdauer von mindestens 12 h mazeriert; und
- den Extrakt und die festen Rückstände des Mazerierungssaftes mit einer Schnitzelgröße von mindestens 0,22 µm insbesondere durch Filtrieren voneinander trennt.

2. Verfahren nach Anspruch 1, **dadurch gekennzeichnet, dass** die verwendete Zichorie-Menge derart ist, dass das Lösungsmittel/Zichorie-Gemisch mindestens 5 %, vorzugsweise 7,4 % Trockensubstanz enthält.

3. Verfahren nach einem der Ansprüche 1 oder 2, **dadurch gekennzeichnet, dass** man vor der Trennung, insbesondere durch Filtration, mit einer Schnitzelgröße von mindestens 0,22 µm eine erste Trennung, insbesondere Filtration, mit einer Schnitzelgröße zwischen 10 und 20 µm durchführt.

4. Verfahren nach Anspruch 3, **dadurch gekennzeichnet, dass** man nach dem ersten Filtrieren das Filtrat so verdünnt, dass die Trockensubstanz des Filtrats 4,5 % oder im Wesentlichen 4,5 % beträgt.

5. Verfahren nach einem der Ansprüche 1 bis 4, **dadurch gekennzeichnet, dass** man die Zichorie vor dem Mazerieren röstet.

6. Verfahren nach einem der Ansprüche 1 bis 5, **dadurch gekennzeichnet, dass** die Zichorie in Form eines Pulvers vorliegt und dass man unter schwachem Rühren eine homogene Mischung aus der Zichorie und dem Lösungsmittel herstellt.

7. Verfahren nach einem der Ansprüche 1 bis 6, **dadurch gekennzeichnet, dass** die Phase der Mazerierung unter Rühren durchgeführt wird.

8. Verfahren nach einem der Ansprüche 1 bis 7, **dadurch gekennzeichnet, dass** man den Mazerierungssaft bei einer Schnitzelgröße von ≤ 10 um abfiltriert.

9. Verfahren nach einem der Ansprüche 1 bis 8, **dadurch gekennzeichnet, dass** man als Lösungsmittel Wasser verwendet.

10. Verfahren nach Anspruch 9, **dadurch gekennzeichnet, dass** man die Zichorie bei einer Temperatur von 4 °C oder im Wesentlichen von 4 °C mazeriert.

11. Verfahren nach Anspruch 10, **dadurch gekennzeichnet, dass** man die Zichorie für eine Zeitsdauer von 24 h oder von im Wesentlichen 24 h mazeriert.

12. Verwendung des bei dem Verfahren nach einem der Ansprüche 1 bis 11 erhaltenen Zichorie-Extrakts zur Herstellung einer Mundpflege-Zusammensetzung.

13. Verwendung des bei dem Verfahren nach einem der Ansprüche 1 bis 11 erhaltenen Zichorie-Extrakts zur Herstellung einer kosmetischen Zusammensetzung.

14. Mundspülmittel, das den bei dem Verfahren nach einem der Ansprüche 2 bis 11 erhaltenen Zichorie-Extrakt in einer Menge zwischen 8 und 12 Massenprozent, vorzugsweise von 10 Massenprozent oder im Wesentlichen von 10 Massenprozent enthält.

15. Zahncreme, die den bei dem Verfahren nach einem der Ansprüche 2 bis 11 erhaltenen Zichorie-Extrakt in einer Menge zwischen 8 und 12 Massenprozent, vorzugsweise von 10 Massenprozent oder im Wesentlichen von 10 Massenprozent enthält.

## Claims

1. A process for the solvent extraction of active compounds from chicory, **characterised in that**:
- chicory is macerated in said solvent at a temperature of between 0°C and 6°C, for a period of at least twelve hours; and
- the extract and solid residues are separated by filtration from the maceration liquor, with a cut-off threshold of at least 0.22 µm.

2. A process according to claim 1, **characterised in that** the amount of chicory used is such that the dry matter content of the mixture of solvent plus chicory is at least 5% and preferably 7.4%.

3. A process according to one of claims 1 or 2, **characterised in that** prior to the separation, namely filtration with a cut-off threshold of at least 0.22 µm, a first separation, namely filtration is carried out with a cut-off threshold of between 10 µm and 20 µm.

4. A process according to claim 3, **characterised in that** after said first filtration, the filtrate is diluted so that the dry matter in said filtrate is equal or approximately equal to 4.5%.

5. A process according to any one of claims 1 to 4, **characterised in that** said chicory is roasted before maceration.

6. A process according to any one of claims 1 to 5, **characterised in that** said chicory is in powder form and **in that** a homogeneous mixture of the chicory and said solvent is prepared with gentle stirring.

7. A process according to any one of claims 1 to 6, **characterised in that** the maceration phase is carried out without stirring.

8. A process according to any one of claims 1 to 7, **characterised in that** the maceration liquor is filtered with a cut-off threshold below or equal to 10 µm.

9. A process according to any one of claims 1 to 8, **characterised in that** said solvent used is water.

10. A process according to claim 9, **characterised in that** said chicory is macerated at a temperature equal or approximately equal to 4°C.

11. A process according to claim 10, **characterised in that** said chicory is macerated for a period equal or approximately equal to 24 hours.

12. Use of the chicory extract obtained by the process according to any one of claims 1 to 11 for the preparation of an oral hygiene composition.

13. Use of the chicory extract obtained by the process according to any one of claims 1 to 11 for the preparation of a cosmetic composition.

14. A mouthwash containing an amount of between 8% and 12% by weight, preferably equal or approximately equal to 10% by weight, of the chicory extract obtained by the process according to any one of claims 2 to 11.

15. A toothpaste containing an amount of between 8% and 12% by weight, preferably equal or approximately equal to 10% by weight, of the chicory extract obtained by the process according to any one of claims 2 to 11.
